# EUROPEAN PATENT SPECIFICATION

(11) **EP 1 676 530 B1**
(45) Date of publication and mention of the grant of the patent: **07.01.2009**
(21) Application number: 05018451.4
(22) Date of filing: 25.08.2005
(51) Int. Cl.: A61B 8/00

(54) **Ultrasound diagnostic system and method for automatically activating a probe**
Ultraschalldiagnosesystem und Methode zur automatischen Aktivierung einer Sonde
Système et méthode de diagnostic à ultra son pour activer automatiquement une sonde

(30) Priority: 29.12.2004 KR 2004114616
(43) Date of publication of application: 05.07.2006
(73) Proprietor: MEDISON CO., LTD., Kangwon-do 250-870 (KR)
(72) Inventor: Lee, Tae Ho, Seoul 138-874 (KR)
(74) Representative: Lorenz, Werner

(56) References cited:
- US-A- 5 505 203
- US-A- 5 552 645
- US-A- 5 718 228
- US-A- 5 776 065
- US-A1- 2002 082 501
- US-A1- 2003 125 629

## Description

The present invention generally relates to an ultrasound diagnostic system, and more particularly to an ultrasound diagnostic system and a method for automatically activating a probe.

An ultrasound diagnostic system generally transmits ultrasound signals to organs and acquires ultrasound images by detecting reflected ultrasound signals from the organs. Particularly, the ultrasound diagnostic system is used to detect foreign materials within a living body, to measure a degree of lesion and to observe, for example, tumors and fetuses.

A conventional ultrasound diagnostic system comprises a plurality of probes, which transmit ultrasound signals to organs and receive ultrasound echo signals from the organs. It also comprises a user input unit and a display unit. In the conventional ultrasound diagnostic system, when a user selects a particular probe to use among various probes through the user input unit, a central controller within the conventional ultrasound diagnostic system controls an operation of the probe selected by the user. However, the conventional,ultrasound system has a problem in that whenever the user selects a probe among various probes, the user must manually input selection information of the probe through the user input unit.

It is an objective of the present invention to provide an ultrasound diagnostic system and a method for automatically activating a probe selected by a user through a sensor mounted on the probe.

In accordance with an aspect of the present invention, there is provided an ultrasound diagnostic system according to claim 1.

In accordance with yet another aspect of the present invention, there is provided a method of automatically activating a probe in an ultrasound diagnostic system according to claim 4.

A system and method according to the preamble of claims 1 and 4 is known from document US-A-5 776 065.

The above and other objects and features of the present invention will become apparent from the following description of preferred embodiments given in conjunction with the accompanying drawings, in which:
Fig. 1 is a front view of an ultrasound diagnostic system in accordance with the present invention;
Fig. 2 is a schematic diagram showing a plurality of probes, a main board and a probe select assembly board provided within a body in the ultrasound diagnostic system shown in Fig. 1; and
Fig. 3 is a flowchart illustrating a process of automatically activating a probe in accordance with the present invention.

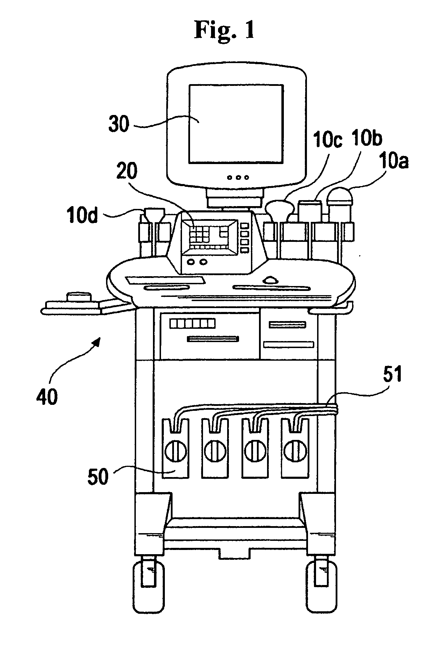

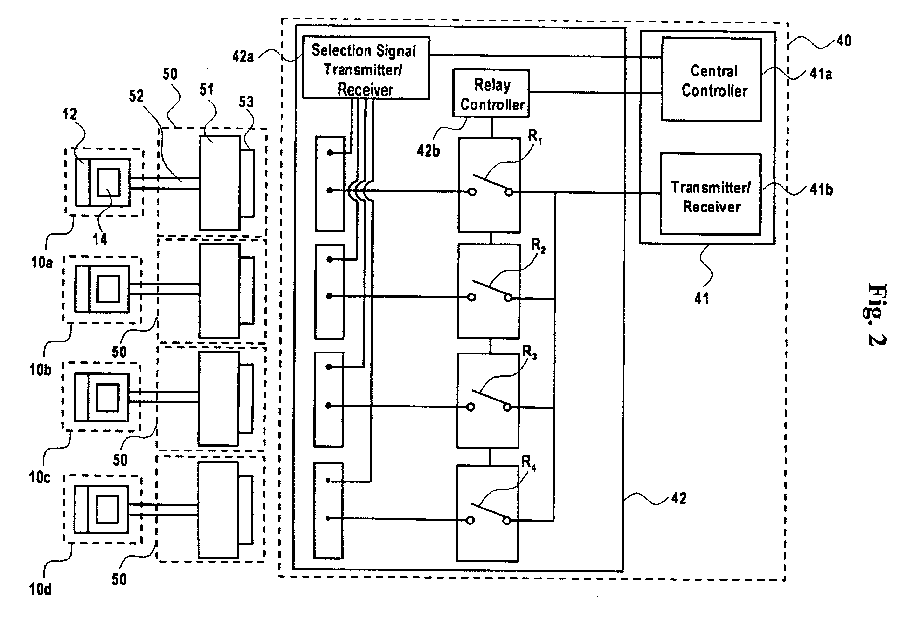

Fig. 1 is a front view of an ultrasound diagnostic system in accordance with the present invention, and Fig. 2 is a schematic diagram showing a plurality of probes, a main board and a probe select assembly board provided within a body in the ultrasound diagnostic system shown in Fig. 1.

Referring to Figs. 1 and 2, the ultrasound diagnostic system in accordance with the present invention comprises: a plurality of probes 10a, 10b, 10c, and 10d, which transmit ultrasound signals to organs and receive ultrasound echo signals from the organs; a user input unit 20; a display unit 30; a body 40; and a connection unit 50 for connecting the probes 10a to 10d to body 40.

Each probe comprises a transducer 12 having a plurality of ultrasound elements and a sensor 14. The sensor 14, which is mounted on each probe 10a to 10d, senses a user touching a probe and generates a selection signal in response thereto. More particularly, when the user touches probe 10a among probes 10a to 10d, the sensor 14 mounted on the probe 10a generates a selection signal. Herein, it is desirable that the sensor 14 can detect the touch of the probe regardless of a position and aspect that the user touches a probe. In the embodiment, a capacitance sensor, a pressure sensor or a temperature sensor can be used as the sensor 14. Further, the sensor 14 may generate the selection signal when the user touches the probes 10a to 10d over a predetermined time, for example, a few seconds.

A body 40 comprises a main board 41 and a Probe Select Assembly (PSA) board 42. The main board 41 comprises a central controller 41a for controlling an activation of the probes and a transmitter/receiver 41b. The main board 41 further comprises a beam former (not shown), an image processor (not shown) and a scan converter (not shown). The PSA board 42 comprises: a selection signal transmitter/receiver 42a for receiving the selection signal generated from the sensor of the selected probe and transmitting the selection signal to the central controller 41a of the main board 41; relays R₁ to R₄ for connecting each probe 10a to 10d with transmitter/receiver 41b; and a relay controller 42b for controlling relays R₁ to R₄ based on a control signal transmitted from the central controller 41a. For example, when the user selects the probe 10a by various ways such as holding or touching the probe 10a, the selection signal transmitter/receiver 42a receives the selection signal from the sensor 14 mounted on the probe 10a and transmits the selection signal to the central controller 41a. The relay controller 42b activates the probe 10a by switching relay R₁ connected to the probe 10a into an on-state, while not activating the probes 10b to 10d by switching relays R₂ to R₄ connected to the probes 10b to 10d into an off-state.

In another embodiment of the present invention, the PSA board 42 may not comprise the selection signal transmitter/receiver 42a. In such a case, the selection signal transmitted from the sensor 14 is directly transmitted to the central controller 41a of the main board 41.

Each connection unit 50 comprises: a connector 51; a first connecting cable 52 for connecting the probe with the connector 51; and a second connecting cable 53 for connecting the connector 51 with the body 40. The connection unit 50 transmits data between the probes 10a to 10d and body 40. The connection unit 50 can be removed when data between the probes 10a to 10d and body 40 is transmitted via a wireless transmitter/receiver.

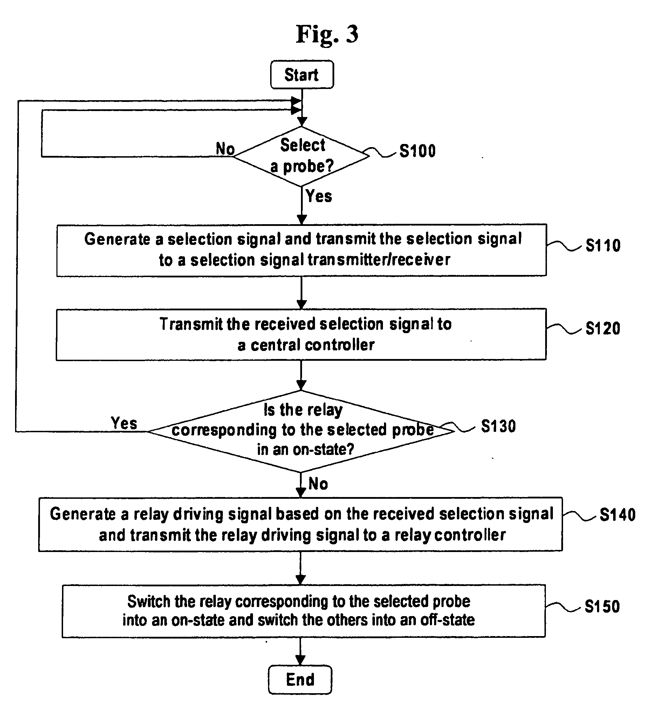

Hereinafter, a process for automatically activating a probe in accordance with the present invention will be described by reference of Figs. 1-3.

The sensor 14 mounted on each probe 10a-10d senses whether a user selects a probe to use at step S100. If it is determined that a probe is not selected, then step S100 is performed. If it is determined that a probe is selected by the user, then the sensor 14 of the probe 10a generates a selection signal and transmits the selection signal to the selection signal transmitter/receiver 42a of the PSA board 42 at step S110.

The selection signal transmitter/receiver 42a transmits the selection signal to the central controller 41 a of the main board 41 at step S120.

The central controller 41 a determines whether a relay corresponding to the selected probe is in an on-state at step S130. If it is determined that the relay is in an on-state, then step S100 is performed to receive a selection of a probe. If it is determined that the relay is in an off-state, central controller 41 a generates a relay driving signal which switches the relay corresponding to the selected probe into an on-state, the others into an off-state, based on the received selection signal, and transmits the relay driving signal to the relay controller 42a at step S 140.

The relay controller 42a switches the relay corresponding to the selected probe to on-state, switches the others to off-state, based on the relay driving signal transmitted from the central controller 41a at step S150.

In another embodiment, two or more sensors may generate the selection signals and transmit the selection signals to the central controller 41 a during a predetermined time. In such a case, the central controller 41a may display an alarm message for informing that a plurality of probes are selected and wait to select one probe. The alarm message may be sound or voice message. In another embodiment, the central controller 41a, which receives the selection signals during a predetermined time, may decide to choose a selection signal among the selection signals based on predetermined superiority, and perform step S140 and step S150 based on the decided selection signal.

As mentioned above, the present invention provides an effect in which a probe can be automatically activated so that a user does not need to input information for selecting a probe through the user input unit. Therefore, convenience can be provided to the user.

While the present invention has been described and illustrated with respect to a preferred embodiment of the invention, it will be apparent to those skilled in the art that variations and modifications are possible without deviating from the broad principles and teachings of the present invention which should be limited solely by the scope of the claims appended hereto.

## Claims

1. An ultrasound diagnostic system, comprising:
a plurality of probes (10a, 10b, 10c, 10d);
a sensor (14) mounted on each of the plurality of probes for sensing a user touching a probe and generating a selection signal in response thereto;
a central controller (41a) for generating a control signal to control an activation of the probe based on the selection signal;
a plurality of relays connected to each of the plurality of probes; **characterized in that** the system further comprises
a relay controller (42b) adapted to switch a relay connected to the touched probe among the plurality of relays into an on-state to activate the touched probe, and to switch the other relays into an off-state not to activate the other probes based on the control signal.

2. The ultrasound diagnostic system as recited in claim 1, wherein the sensor (14) is a capacitance sensor, a pressure sensor or a temperature sensor.

3. The ultrasound diagnostic system as recited in claim 1, wherein the ultrasound diagnostic system further comprises a selection signal transmitting/receiving means (42a) for receiving the selection signal from the sensor and transmitting the selection signal to the central controller.

4. A method of automatically activating a probe in an ultrasound diagnostic system including a plurality of probes, a sensor mounted on each of the plurality of probes, a plurality of relays connected to each of the plurality of probes, and a relay controller, comprising the steps of:
a) at the sensor, sensing a user touching a probe and generating a selection signal in response thereto;
b) at the central controller, generating a control signal based on the selection signal;
**characterized in that** the method further comprises the step of:
c) at the relay controller, switching a relay corresponding to the selected probe to on-state, and switching the others to off-state.

## Patentansprüche

1. Ultraschalldiagnosesystem, welches Folgendes aufweist:
eine Vielzahl von Sonden (10a, 10b, 10c, 10d); einen Sensor (14), der auf jeder der Vielzahl von Sonden angebracht ist, zum Sensieren eines Benutzers, der eine Sonde berührt, und zum Erzeugen eines Auswahlsignals als Reaktion darauf;
ein zentrales Steuergerät (41a) zum Erzeugen eines Steuersignals, um eine Aktivierung der Sonde basierend auf dem Auswahlsignal zu steuern;
eine Vielzahl von Relais, die mit jeder der Vielzahl von Sonden verbunden sind;
**dadurch gekennzeichnet, dass**
das System des weiteren Folgendes aufweist:
eine Relaissteuerung (42b) welche in der Lage ist, ein mit der berührten Sonde verbundenes Relais unter der Vielzahl von Relais in einen eingeschalteten Zustand zu schalten, um die berührte Sonde zu aktivieren, und die anderen Relais in einen ausgeschalteten Zustand zu schalten, um die anderen Sonden basierend auf dem Steuersignal nicht zu aktivieren.

2. Ultraschalldiagnosesystem nach Anspruch 1, wobei der Sensor (14) ein kapazitiver Sensor, ein Drucksensor oder ein Temperatursensor ist.

3. Ultraschalldiagnosesystem nach Anspruch 1, wobei das Ultraschalldiagnosesystem des weiteren eine Auswahlsignal-Übertragungs-/Empfangseinrichtung (42a) zum Empfangen des Auswahlsignals von dem Sensor und Übertragen des Auswahlsignals zu dem zentralen Steuergerät aufweist.

4. Verfahren zur automatischen Aktivierung einer Sonde in einem Ultraschalldiagnosesystem mit einer Vielzahl von Sonden, einem auf jeder der Vielzahl von Sonden angebrachten Sensor, einer Vielzahl mit jeder der Vielzahl von Sonden verbundener Relais und einer Relaissteuerung, welches die folgenden Schritte aufweist:
a) an dem Sensor Sensieren eines Benutzers, welcher eine Sonde berührt, und Erzeugen eines Auswahlsignals als Antwort darauf;
b) an dem zentralen Steuergerät Erzeugen eines Steuersignals basierend auf dem Auswahlsignal;
**dadurch gekennzeichnet , dass** das Verfahren des weiteren den folgenden Schritt aufweist:
c) an der Relaissteuerung Schalten eines Relais entsprechend der ausgewählten Sonde in einen eingeschalteten Zustand und Schalten der anderen in einen ausgeschalteten Zustand.

## Revendications

1. Système de diagnostic à ultrasons, comprenant :
une pluralité de sondes (10a, 10b, 10c, 10d);
un capteur (14) monté sur chacune de la pluralité de sondes pour détecter un utilisateur touchant une sonde et pour générer un signal de sélection en réponse à cela;
un contrôleur central (41a) pour générer un signal de commande pour commander une activation de la sonde basée sur le signal de sélection;
une pluralité de relais reliés à chacune de la pluralité de sondes, **caractérisé en ce que** le système comprend en outre :
un contrôleur de relais (42b) agencé pour faire basculer un relais relié à la sonde touchée parmi la pluralité de relais dans un état passant pour activer la sonde touchée, et pour faire basculer les autres relais dans un état bloqué pour ne pas activer les autres sondes basées sur le signal de commande .

2. Système de diagnostic à ultrasons tel qu'énoncé dans la revendication 1, dans lequel le capteur (14) est un capteur capacitif, un capteur de pression ou un capteur de température.

3. Système de diagnostic à ultrasons tel qu'énoncé dans la revendication 1, dans lequel le système de diagnostic à ultrasons comprend en outre des moyens de réception/transmission (42a) d'un signal de sélection pour recevoir le signal de sélection du capteur et transmettre le signal de sélection au contrôleur central.

4. Procédé d'activation automatique d'une sonde dans un système de diagnostic à ultrasons comportant une pluralité de sondes, un capteur monté sur chacune de la pluralité de sondes, une pluralité de relais reliés à chacune de la pluralité de sondes, et un contrôleur de relais, comprenant les étapes de :
a) u niveau du capteur, détection d'un utilisateur touchant une sonde et génération d'un signal de sélection en réponse à cela;
b) u niveau du contrôleur central, génération d'un signal de commande basé sur le signal de sélection;
**caractérisé en ce que** le procédé comprend en outre l'étape de:
c) u niveau du contrôleur de relais, basculement d'un relais correspondant à la sonde choisie en un état passant, et basculement des autres dans un état bloqué.
